# EUROPEAN PATENT SPECIFICATION

(11) **EP 2 878 682 B1**
(45) Date of publication and mention of the grant of the patent: **06.07.2016**
(21) Application number: 13194538.8
(22) Date of filing: 27.11.2013
(51) Int. Cl.: C12R 1/865, A23L 33/135, A23L 2/38, C12R 1/02, A23L 2/52, A23L 2/56, A23L 2/66, C12R 1/01

(54) **Association LMKK P1398 and method for obtaining fermented non-alcoholic beverages**
Zuordnungs-LMK P1398 und Verfahren zur Herstellung fermentierter nichtalkoholischer Getränke
Association LMKK P1398 et procédé pour obtenir des boissons non alcoolisées fermentées

(43) Date of publication of application: 03.06.2015
(73) Proprietor: Latvijas Universitate, 1586 Riga (LV)
(72) Inventor: Semjonovs, Pavels, LV-1024 Riga (LV); Danilevics, Aleksejs, LV-1079 Riga (LV); Auzina, Lilija, LV-1055 Riga (LV); Denina, M Ilze, LV-4401 Gulbene (LV); Upitis, Andris, LV-5000 Ogre (LV)
(74) Representative: Fortuna, Aleksandra

(56) References cited:
- EP-A1- 0 791 296
- JP-A- 2006 124 290
- US-A1- 2007 116 801

## Description

### Field of invention

The invention is related to food biotechnology and can be used for obtaining of fermented polyfunctional synbiotic beverages for mass consumption.

### Background art

There are known natural microorganism associations that are used for the obtaining of fermented beverages. For instance Tea fungus or Kombucha, Waterkefir or Tibi, Tibetan milk mushroom and kefir zoogloeas belong to such associations.

Zoogloeas of these microbial associations consist of various microorganisms, mainly different lactic acid and acetic acid bacteria and yeasts. For example in literature [1] is given such composition of Tea fungus natural microorganism association: acetic acid bacteria *Acetobacter xylinum, Acetobacter xylinoides* and yeasts *Schizosaccharomyces pombe, Saccharomyces ludwigii, Zygosaccharomyces rouxii, Candida sp., Pichia membranefaciens.* According to another source [2] other yeast species were isolated from Tea fungus natural microorganism association - *Zygosaccharomyces bailii, Torulaspora delbreuckii, Rhodotorula mucilaginosa, Brettanomyces bruxellensis and Candida stellata.* Yeast *Saccharomyces cerevisiae* and lactic acid bacteria *Lactobacillus brevis* and *Streptococcus lactis* were isolated from Waterkefir association [3]. According to [4] lactic acid bacteria *Lactobacillus hordei*, *Lactobacillus nagelii, Lactobacillus casei, Leuconostoc mesenteroides, Lactobacillus citreum, Lactobacillus hilgardii* and yeasts *Saccharomyces cerevisiae, Lachancea fermentati, Hanseniaspora sp., Zygotorulaspora florentina* were found in this association.

Since olden days natural microorganism associations of zoogloeas have been used for the production of fermented beverages for household use. Fermentation liquids obtained by zoogloeas of natural microorganism associations have rich chemical composition. Zoogloeas of natural microorganism associations produce various biologically active organic acids, like acetic, lactic, succinic, gluconic, malic, citric acid and others during the fermentation. In addition mono and disaccharide residues remain in the liquid, small amount of ethanol is produced, vitamins and other biologically active substances are produced.

Whole complex of produced substances define biological value of the beverage and its organoleptic properties.

Use of natural microorganism associations' zoogloeas, their parts or parts of fermentation broth (as inoculums) is characteristic for current production of fermented refreshing beverages. Thus it is not possible to provide stability of constituents of microbial association, replicability and stability of technological process; it is not possible to warrant microbial safety of obtained beverage and its standardized quality; it is not possible to mechanize several stages of technological process that essentially raise the costs of the technological process and restricts production yield.

Therefore it is more convenient to use prepared beforehand starter with defined artificially selected microorganism composition not producing zoogloea for the production of beverage. It allows obtaining healthy and tasty beverage during fast fermentation process. This kind of starter should also provide stable microbial composition and constant quality of various beverage batches.

EP 0791296A1 discloses a fermented beverage made by fermenting tea or coffee infusions with cultures containing Saccharomyces cerevisiae Castelli strain and Gluconobacter suboxydans strain DSM 50049. JP 2006 124290A discloses an association of cultures comprising a number of strains including Gluconobacter oxydans SIID1719-3b and Saccharomyces cerevisiae SIID1719-4y strains.

### Disclosure of invention

Starter culture for obtaining fermented polyfunctional beverage - symbiotic association consisting of acetic acid bacteria and yeast was isolated from natural microbial association. Role of individual cultures of microbial association in development of properties of fermented beverage was evaluated during the selection of starter cultures. Microbiologically defined symbiotic acetic acid bacteria and yeast association and individual cultures of it do not form zoogloea that essentially simplify technological process and allows obtaining microbiologically safe beverages with standardized chemical composition and constant organoleptic properties. Application of association allows essentially speed up fermentation process, reducing its duration down to 4-48 hours in comparison with 4-14 days that is necessary for obtaining beverage of good quality by use of zoogloeas forming natural microbial associations. Yeast isolates were identified in National Collection of Agricultural and Industrial Microorganisms, Faculty of Food

Sciences, Corvinus University of Budapest. Isolates of bacteria were identified at Bacterial Collection of Microbiological Laboratory, Gent University (BCCM/LMG). Starter culture association that consists of individual cultures *Saccharomyces cerevisiae* LUMBI R23 and *Gluconobacter sp.* LUMBI B32 in ratio 3:1 were deposited in Latvian Culture Collection of Microorganisms under accession number P 1398.

Constituent cultures of association LMKK P 1398 were identified as: (1) yeast *Saccharomyces cerevisiae* LUMBI R23; (2) acetic acid bacteria *Gluconobacter sp.* LUMBI B32.

Characterisation of association LMKK P 1398 constituent cultures:

### Saccharomyces cerevisiae

White to cream, butyrous colonies. Vegetative reproduction by budding. Do not form filaments. Persistent asci containining up to 4 oval or round spores on McClary acetate agar at +25°C. Ferment D-glucose. Grow at +25 and +30° C, do not grow at +45°C. Do not grow in 60% glucose and 16% NaCl. Do not form starch, do not produce acetic acid, and do not hydrolyze urea. Do not ferment: lactose, cellobiose, D-xylose. Grow on: glucose. Do not grow on: L-sorbose, D-glucosamine, D-ribose, D-xylose, D-arabinose, L-arabinose, L-rhamnose, cellobiose, salicin, arbutin, lactose, inulin, erythritol, ribitol, xylitol, L-arabinitol, D-glucitol, D-mannitol, D-galactitol, myoinositol, D-glucono-1,5-D-lactone, 2-keto-D-gluconate, 5-keto-D-gluconate, D-gluconate, D-glucuronate, D-galacturonic acid, succinate, citrate, methanol, propane-1,2-diol, 2,3-butandiol, nitrites nitrates.

### Gluconobacter sp.

Nonmotile rods, gram-negative. Optimal growth temperature + 30°C. Oxidize ethanol to acetic acid containing medium, do not oxidize acetic acid and lactate to CO₂ and H₂O. Grow on 0.35% acetic acid media with. Do not grow in presence of 30% glucose. Do not grow on methanol. Grow on mannitol. Do not synthesize cellulose. Produce dihydroxyacetone from glycerol. Produce acid from mannitol, glycerol, do not produce from raffinose.

It is proposed to apply microbial culture association LMKK P 1398 as a starter culture for production of fermented beverages. Association LMKK P 1398 should be cultured at +15+40 °C, fermentation medium should contain 1- 300 g/l carbohydrates or carbohydrates containing raw materials. Malt, molasses, honey, fruit or vegetable juices, fruit or vegetable juice concentrates can be used as carbohydrates containing medium or carbohydrates containing raw materials. Medium can be supplemented wit dried or fresh fruits, berries or vegetables, plant premixes, their concentrates or extracts.

### Examples of application

### Example 1

Example illustrates fermentation of sucrose containing medium by association LMKK P 1398. Fermentation medium consists of decocted tea (4 g/l), sucrose 7%, inoculum 2.3 g/l. Inoculum consists of acetic acid bacteria *Gluconobacter sp.* LUMBI B32 and yeast *Saccharomyces cerevisiae* LUMBI R23 in ratio 3:1. Fermentation is performed at +30° C during 24 - 48 hours, in incubation shaker (rmp 180, orbit 2.54 cm). Zoogloea has not been formed during the fermentation. Sensory properties of obtained beverage were evaluated as good; physically-chemical properties are depicted in Table 1.

**Table 1. Formula of fermented beverage obtained by association LMKK P 1398 and its physically-chemical properties**

| **Parameter** | **Parameter's indices** | | |
|---|---|---|---|
| Composition and amount of inoculum | *Gluconobacter sp. LUMBI B32 : Saccharomyces cerevisiae* LUMBI R23 in ratio 3:1; 2.3g/l | | Tea fungus natural microbial association |
| Fermentation medium | Sucrose 7%, black tea 4 g/l | | Sucrose 7%, black tea 4 g/l |

| | Duration of fermentation | | Duration of fermentation |
|---|---|---|---|
| | 24 hours | 48 hors | 48 hours |
| Biomass g/l | 0.16 | 1.38 | - |
| pH | 2.27 | 2.10 | 5.5 |
| Titratable acidity, °T | 18 | 75 | 12 |
| Sucrose, % | 4.0 | 2.23 | 65.20 |
| Citric acid, mg/ml | 0.87 | 1.46 | 0.05 |
| Malic acid, mg/ml | 0.09 | 0.13 | 0.05 |
| Succinic acid, mg/ml | 0.27 | 0.29 | 0.09 |
| Formic acid, mg/ml | 0.02 | signs | signs |
| Acetic acid, mg/ml | 0.17 | 0.24 | 0.04 |
| Formation of zoogloea | No | No | Yes |
| Aeration | Yes | Yes | No |

Unlike natural tea fungus association, association LMKK P 1398 do not form zoogloea, it is cultivated with aeration or mixing (200 rpm/min), duration of fermentation with this association is substantially shorter (48 h) in comparison with natural association (7-14 days). Applying zoogloea forming natural association, fermented beverage is not ready yet after 48 h (Table 1).

Obtained results show that not only sensory properties of obtained beverage were highly evaluated but it also contains biologically valuable organic acids.

### Example 2

Example illustrates fermentation of glucose or/and fructose containing medium by association LMKK P 1398. The starter culture consists of *Gluconobacter sp.* LUMBI B32 and *Saccharomyces cerevisiae* LUMBI R2 in ratio 3:1. Fermentation medium consists of decocted tea with addition of glucose or fructose or their mixture in ratio 1:1 as carbon sources, inoculated with 2.3g/l starter culture. Fermentation was performed under the same conditions as in Example 1. Sensory properties of obtained beverage were evaluated as pleasant. Physically-chemical properties are shown in Table 2.

**Table 2. Composition of fermented beverage obtained by association LMKK P 1398 and its physically-chemical properties**

| **Parameter** | **Parameter's indices** | | | | | |
|---|---|---|---|---|---|---|
| Fermentation medium | 5% glucose; 7% fructose; 7% glucose and fructose, tea 0.4% | | | | | |
| Composition and amount of inoculum | *Gluconobacter sp. LUMBI B32: Saccharomyces cerevisiae* LUMBI R23 in ratio 3:1; 2.3g/l | | | | | |
| | Carbon sources | | | | | |
| | 5% glucose | | 7% fructose | | 3.5% glucose and 3.5% fructose (1:1) | |

| | Duration of fermentation, hours | | | | | |
|---|---|---|---|---|---|---|
| | **24** | **48** | **24** | **48** | **24** | **48** |
| Biomass g/l | 0.21 | 0.27 | 0.45 | 0.54 | 0.27 | 0.30 |
| Titratable acidity, ⁰T | 208 | 276 | 20 | 28 | 182 | 204 |
| pH | 1.70 | 1.66 | 3.6 | 3.46 | 1.74 | 1.72 |
| Oxalic acid, mg/ml | 0.028 | 0.036 | signs | signs | 0.01 | 0.01 |
| Citric acid, mg/ml | 1.11 | 1.29 | 0.29 | 0.35 | 0.80 | 0.82 |
| Gluconic acid, mg/ml | 18.39 | 20.02 | 4.93 | 9.64 | 14.02 | 15.65 |
| Malic acid, mg/ml | - | - | 4.75 | 4.20 | 5.19 | 5.95 |
| Succinic acid, mg/ml | 0.13 | 0.15 | 0.45 | 0.47 | 0.17 | 0.18 |
| Formic acid, mg/ml | 0.00 | 0.00 | 0.02 | 0.47 | 0.00 | 0.00 |
| Acetic acid, mg/ml | 0.41 | 0.37 | 0.89 | 1.23 | 0.63 | 0.84 |
| Ethanol, mg/ml | 0.01 | 0.02 | 0.02 | 0.03 | 0.03 | 0.03 |

Beverage with most pleasant sensory properties is obtained by use of glucose and fructose mixture (1:1) as carbon source for the fermentation by association LMKK P 1398. Formation of zoogloea was not observed.

### References

1. Chen C., Lin B.I. (2000) Changes in major components of tea fungus metabolites during prolonged fermentation. J Appl Microbiol, 89:834-839.
2. Teoh A.L, Heard G., Cox J. (2004) Yeast ecology of Kombucha fermentation. Int J Food Microbiol, 95:119-126.
3. Reiβ J. (1990) Metabolic activity of Tibi grains. Lebensm Unters Forsch, 191:462-465.
4. Gulitz A., Stadie I., Wenning M., Ehrman M.A., Vogel R.E. (2011) The microbial diversity of water kefir. Int J Food Microbiol, 151:284-288.

## Claims

1. An association of microbial cultures consisting of individual cultures *Gluconobacter sp.* LUMBI B32 and *Saccharomyces cerevisiae* LUMBI R23 in ratio 3:1 deposited in Latvian Culture Collection of Microorganisms under accession number P 1398.

2. Use of association in accordance to claim 1 as a starter culture for the production of fermented beverages.

3. A method for obtaining fermented beverages using the association in accordance with claim 1, where fermentation is performed at +15+40 °C, fermentation medium containing carbohydrates or carbohydrates containing raw materials 1- 300 g/l.

4. The method according to claim 3, where medium containing carbohydrates or raw materials containing carbohydrates are malt, molasses, honey, fruit juice, vegetable juice, fruit juice concentrate or vegetable juice concentrate.

5. The method according to claim 3 or 4, where fermentation medium is supplemented with dried or fresh fruits, berries or vegetables, plant premixes, their concentrates or extracts.

## Patentansprüche

1. Eine Sammlung mikrobieller Kulturen, die aus einzelnen Kulturen des *Gluconobacter sp. LUMBI B32 und* Saccharomyces cerevisiae *LUMBI R23* im Verhältnis 3:1 besteht und in einer lettischen Kultursammlung von Mikroorganismen unter der Zugangsnummer P 1398 aufbewahrt wird.

2. Verwendung der Sammlung gemäß Anspruch 1 als Starterkultur für die Herstellung fermentierter Getränke.

3. Verfahren zur Herstellung fermentierter Getränke, bei der die Sammlung gemäß Anspruch 1 verwendet und die Fermentierung bei +15 +40 °C durchgeführt wird, und das Fermentationsmedium Kohlenhydrate oder rohstoffhaltige Kohlenhydrate in einer Menge von 1- 300 g/l enthält.

4. Verfahren nach Anspruch 3, bei dem das Medium, das Kohlenhydrate oder rohstoffhaltige Kohlenhydrate enthält aus Malz, Melasse, Honig, Fruchtsaft , Gemüsesaft, Fruchtsaftkonzentrat oder Gemüsesaftkonzentrat besteht.

5. Verfahren nach Anspruch 3 oder 4, in dem das Fermentationsmedium durch getrocknete oder frische Früchte, Beeren oder Gemüse, Pflanzvormischungen ihrer Konzentrate oder Extrakte ergänzt wird.

## Revendications

1. Association de cultures microbiennes constituée de cultures individuelles *Glarconobacter sp.* LUMBI B32 et *Saccharomyces cerevisiae* LUMBI R23 dans un rapport 3:1 déposées à la collection de cultures de micro-organismes lettone (Latvian Culture Collection of Microorganisms) sous le numéro d'accession P 1398.

2. Utilisation de l'association selon la revendication 1 en tant que culture de départ pour la production de boissons fermentées.

3. Procédé d'obtention de boissons fermentées en utilisant l'association selon la revendication 1, où la fermentation est effectuée à +15+40 °C, le milieu de fermentation contenant des glucides ou des matières premières contenant des glucides à raison de 1 à 300 g/L.

4. Procédé selon la revendication 3, où le milieu contenant des glucides ou les matières premières contenant des glucides sont du malt, de la mélasse, du miel, du jus de fruits, du jus de légumes, un concentré de jus de fruits ou un concentré de jus de légumes.

5. Procédé selon la revendication 3 ou 4, où le milieu de fermentation est complété avec des fruits, baies ou légumes, séchés ou frais, des prémélanges de plantes, leurs concentrés ou extraits.
